# EUROPEAN PATENT SPECIFICATION

(11) **EP 3 019 183 B1**
(45) Date of publication and mention of the grant of the patent: **02.09.2020**
(21) Application number: 14738387.1
(22) Date of filing: 07.07.2014
(51) Int. Cl.: A61K 36/899, A61K 36/18, A61K 36/28, A61K 36/66, A61K 36/73, A61P 25/00, A61P 25/28, A61K 36/185, A61K 36/53, A61K 36/534

(54) **REUPTAKE INHIBITORS**
INHIBITOREN DER NEUROTRANSMITTERWIEDERAUFNAHME
INHIBITEURS DE RE-CAPTAGE DE NEUROTRANSMETTEURS

(30) Priority: 09.07.2013 EP 13003468
(43) Date of publication of application: 18.05.2016
(60) Divisional application: 20170788.2
(73) Proprietor: Pandalis, Georgios, 49219 Glandorf (DE)
(72) Inventor: Pandalis, Georgios, 49219 Glandorf (DE)
(74) Representative: Grünecker Patent- und Rechtsanwälte PartG mbB
(86) International application number: PCT/EP2014/001856
(87) International publication number: WO 2015/003793

(56) References cited:
- WO-A1-2013/087217
- LYDIA KAUME ET AL: "The Blackberry Fruit: A Review on Its Composition and Chemistry, Metabolism and Bioavailability, and Health Benefits", JOURNAL OF AGRICULTURAL AND FOOD CHEMISTRY, vol. 60, no. 23, 13 June 2012 (2012-06-13) , pages 5716-5727, XP055074008, ISSN: 0021-8561, DOI: 10.1021/jf203318p
- SHUKITT-HALE BARBARA ET AL: "Effects of blackberries on motor and cognitive function in aged rats.", NUTRITIONAL NEUROSCIENCE JUN 2009, vol. 12, no. 3, June 2009 (2009-06), pages 135-140, XP009171765, ISSN: 1476-8305
- CHERNIACK E PAUL: "A berry thought-provoking idea: the potential role of plant polyphenols in the treatment of age-related cognitive disorders.", THE BRITISH JOURNAL OF NUTRITION SEP 2012, vol. 108, no. 5, September 2012 (2012-09), pages 794-800, XP009171713, ISSN: 1475-2662
- LUCÉLIA TAVARES ET AL: "Neuroprotective effects of digested polyphenols from wild blackberry species", EUROPEAN JOURNAL OF NUTRITION, STEINKOPFF-VERLAG, DA, vol. 52, no. 1, 8 February 2012 (2012-02-08), pages 225-236, XP035168195, ISSN: 1436-6215, DOI: 10.1007/S00394-012-0307-7
- LUCLIA TAVARES ET AL: "Neuroprotective effect of blackberry (sp.) polyphenols is potentiated after simulated gastrointestinal digestion", FOOD CHEMISTRY, ELSEVIER LTD, NL, vol. 131, no. 4, 10 October 2011 (2011-10-10), pages 1443-1452, XP028118867, ISSN: 0308-8146, DOI: 10.1016/J.FOODCHEM.2011.10.025 [retrieved on 2011-10-15]
- POULOSE S M ET AL: "Improving brain signaling in aging: Could berries be the answer?", EXPERT REVIEW OF NEUROTHERAPEUTICS 2012 EXPERT REVIEWS LTD. GBR, vol. 12, no. 8, August 2012 (2012-08), pages 887-889, XP002709638, ISSN: 1473-7175
- TIRGAR P R ET AL: "Investigation into beneficial effects of triticum aestivum (wheat grass) in iron overload complications", PHARMACOLOGYONLINE 2011 UNIVERSITY OF SALERNO ITA, vol. 2, 2011, pages 900-920, XP009171717, ISSN: 1827-8620
- AHMED N: "Alloxan diabetes-induced oxidative stress and impairment of oxidative defense system in rat brain: Neuroprotective effects of cichorium intybus", INTERNATIONAL JOURNAL OF DIABETES AND METABOLISM, INTERNATIONAL JOURNAL OF DIABETES, SE, vol. 17, no. 3, 1 January 2009 (2009-01-01), pages 105-109, XP009151136, ISSN: 1606-7754
- AHMED NAYEEMUNNISA ET AL: "Acetylcholinesterase activity in the brain of alloxan diabetic albino rats: Presence of an inhibitor of this enzyme activity in the cerebral extract.", INTERNATIONAL JOURNAL OF DIABETES IN DEVELOPING COUNTRIES OCT 2009, vol. 29, no. 4, October 2009 (2009-10), pages 174-177, XP002709640, ISSN: 1998-3832
- SUNIL D. KULKARNI ET AL: "Evaluation of the antioxidant activity of wheatgrass (Triticum aestivum L.) as a function of growth under different conditions", PHYTOTHERAPY RESEARCH, vol. 20, no. 3, 1 March 2006 (2006-03-01), pages 218-227, XP055074013, ISSN: 0951-418X, DOI: 10.1002/ptr.1838
- MIYAZAWA M ET AL: "INHIBITION OF ACETYLCHOLINESTERASE ACTIVITY BY ESSENTIAL OILS OF MENTHA SPECIES", JOURNAL OF AGRICULTURAL AND FOOD CHEMISTRY, AMERICAN CHEMICAL SOCIETY, US, vol. 46, no. 9, 1 September 1998 (1998-09-01), pages 3431-3434, XP000776608, ISSN: 0021-8561, DOI: 10.1021/JF9707041
- Broadhurst C.L. and Duke J.A.: "Preventing Alzheimer's with Herbs", , 31 July 1999 (1999-07-31), XP002730978, Retrieved from the Internet: URL:http://www.motherearthliving.com/healt h-and-wellness/inside-plants-rosemary-comp ounds-help-the-brain-manage-memory-16-rose mary-compounds-help-the-.aspx#axzz3FkblUvd 7 [retrieved on 2014-10-10]
- MUSHTAQ M ET AL: "Polyphenols and human health- A review", INTERNATIONAL JOURNAL OF PHARMA AND BIO SCIENCES 2013 INTERNATIONAL JOURNAL OF PHARMA AND BIO SCIENCES IND, vol. 4, no. 2, June 2013 (2013-06), pages B338-B360, XP009171716, ISSN: 0975-6299
- OLSEN H T ET AL: "Isolation of the MAO-inhibitor naringenin from Mentha aquatica L", JOURNAL OF ETHNOPHARMACOLOGY, ELSEVIER IRELAND LTD, IE, vol. 117, no. 3, 22 May 2008 (2008-05-22), pages 500-502, XP022635284, ISSN: 0378-8741, DOI: 10.1016/J.JEP.2008.02.015 [retrieved on 2008-02-19]
- POURMOTABBED A ET AL: "Effects of Papaver rhoeas extract on the expression and development of morphine-dependence in mice", JOURNAL OF ETHNOPHARMACOLOGY,, vol. 95, no. 2-3, 1 December 2004 (2004-12-01), pages 431-435, XP004990600, ISSN: 0378-8741, DOI: 10.1016/J.JEP.2004.08.022
- Monica Rosa Loizzo ET AL: "Chemistry and functional properties in prevention of neurodegenerative disorders of five Cistus species essential oils", FOOD AND CHEMICAL TOXICOLOGY., vol. 59, 2 July 2013 (2013-07-02), pages 586-594, XP55595228, GB ISSN: 0278-6915, DOI: 10.1016/j.fct.2013.06.040
- OLSEN H T ET AL: "Isolation of the MAO-inhibitor naringenin from Mentha aquatica L", JOURNAL OF ETHNOPHARMACOLOGY, ELSEVIER IRELAND LTD, IE, vol. 117, no. 3, 22 May 2008 (2008-05-22), pages 500-502, XP022635284, ISSN: 0378-8741, DOI: 10.1016/J.JEP.2008.02.015 [retrieved on 2008-02-19]
- Anonymous ET AL: "Assessment report on Cichorium intybus L., radix", , 15 January 2013 (2013-01-15), XP55576996, Retrieved from the Internet: URL:https://www.ema.europa.eu/en/documents /herbal-report/final-assessment-report-cic horium-intybus-l-radix_en.pdf [retrieved on 2019-04-03]
- Melanie-Jayne R Howes ET AL: "Acetylcholinesterase Inhibitors of Natural Origin", International Journal of Biomedical and Pharmaceutical Sciences, 1 January 2009 (2009-01-01), XP55595426, Retrieved from the Internet: URL:http://www.globalsciencebooks.info/Onl ine/GSBOnline/images/0906/IJBPS_3(SI1)/IJB PS_3(SI1)67-86o.pdf [retrieved on 2019-06-11]
- CHAIYANA W ET AL: "Characterization of potent anticholinesterase plant oil based microemulsion", INTERNATIONAL JOURNAL OF PHARMACEUTICS, ELSEVIER, NL, vol. 401, no. 1-2, 30 November 2010 (2010-11-30), pages 32-40, XP027450380, ISSN: 0378-5173, DOI: 10.1016/J.IJPHARM.2010.09.005 [retrieved on 2010-10-28]
- S. Saeed-Abadi ET AL: "Effects of Papaver rhoeas (L.) extract on formalin-induced pain and inflammation in mice.", Pakistan Journal of Biological Sciences, vol. 15, no. 21, 1 January 2012 (2012-01-01), pages 1041-1044, XP55595786,

## Description

The present invention relates to compositions for use in inhibiting the reuptake of serotonin and inhibiting acetylcholinesterase.

Neurotransmitters are chemicals that transmit signals from a neuron to a target cell across a synapse. Neurotransmitters are packaged into synaptic vesicles clustered beneath the membrane in the axon terminal, on the presynaptic side of a synapse. They are released into and diffuse across the synaptic cleft, where they bind to specific receptors in the membrane on the postsynaptic side of the synapse.

Common neurotransmitters are amino acids, such as glutamate, aspartate, serine, γ-amino butyric acid, glycine, monoamines and other biogenic amines, such as dopamine, norepinephrine, epinephrine, histamine, serotonin, peptides, such as somatostatin, substance P, opioid peptides. Further neurotransmitters are acetylcholin, , adenosine, anandamide, and nitric oxide.

A neurotransmitter activates one or more types of receptors. The effect on the postsynaptic cell depends entirely on the properties of those receptors, and the receptor may have excitatory or inhibitory effects.

For example, dopamine has a number of important functions in the brain, e.g. regulation of motor behaviour, pleasures related to motivation and emotional arousal. People with Parkinson's disease have been linked to low levels of dopamine and people with schizophrenia have been linked to high levels of dopamine. Serotonin is a monoamine neurotransmitter and functions to regulate appetite, sleep, memory, learning, temperature, mood, behaviour, muscle contraction, and functions of the cardiovascular system and endocrine system. It is speculated to have a role in depressions.

Drugs targeting the neurotransmitter of such systems affect the whole system. Diseases may affect specific neurotransmitter systems. For instance, Parkinson's disease is at least in part related to failure of dopaminergic cells in deep-brain nuclei, for example the substantia nigra. Levodopa is a precursor of dopamine and is the most widely used drug to treat Parkinson's disease.

A neurotransmitter must be broken down once it reaches the postsynaptic cell to prevent further excitatory or inhibitory signal transduction. For example, acetylcholine, an excitatory neurotransmitter, is broken down by acetylcholinesterase. Dopamine is able to diffuse away from the targeted synaptic junctions and is eliminated from the body via the kidneys or destroyed in the liver.

The synaptic actions of most neurotransmitters are inactivated by reuptake into the nerve terminals from which they are released, or by uptake into adjacent cells. This process is mediated by a family of transporter molecules. A reuptake inhibitor, also known as transporter blocker, inhibits the plasmalemmal transporter-mediated reuptake of a neurotransmitter from the synapse in the presynaptic neuron, leading to an increase in the extracellular concentrations of the neurotransmitter and therefore an increase in neurotransmission. Reuptake inhibitors bind directly to the respective transporter of the neurotransmitter and occupy the transporter. The transporter will then be less efficient at removing the neurotransmitter from the synapse. The transporters for norepinephrine and serotonin are key targets for antidepressant drugs. Norepinephrine-selective and serotonin-selective reuptake inhibitors are effective against major depressions and a range of other psychiatric illnesses.

Furthermore, an inhibitor of acetylcholinesterase inhibits the enzyme and less acetylcholine is hydrolyzed, which also leads to a higher concentration of acetylcholine in the synaptic cleft.

The level of a neurotransmitter, e.g. serotonin, can be controlled by different ways. One possibility for controlling the serotonin level is to increase the level of 5-hydroxytryptophan (5-HPT). 5-HPT is a prodrug from which serotonin can be synthesized. Alternatively, the serotonin, dopamine and norepinephrine level can be controlled by monoaminoxidase inhibitors (MAO inhibitors), e.g. tricyclic antidepressants. As a further alternative, selective or non-selective reuptake inhibitors can be used. Tricyclic antidepressant inhibit monoamine uptake mostly non-selective. A selective reuptake inhibitor is able to selectively inhibit the reuptake of the respective neurotransmitter.

As selective reuptake inhibitor of serotonin (SSRI) a number of synthetic compounds are known, e.g. fluvoxamin, fluoxetin, and paroxetin. However, such substances may have serious side effects, especially in view of the fact that such a medicamentation is taken over a long period of time.

Another therapeutic form for treating diseases is phytotherapy. In phytotherapy plants and plant parts are used for treating the disease. The plants or plant parts are administered in form of teas, juices, tinctures, powders, decoctions and extracts. Extracts from St. John's wort (*Hypericum perforatum*) are known for long to act as serotonin reuptake inhibitor. However, when given in high doses, it may cause photosensitivity. *Sceletium tortuosum* also acts as serotonin reuptake inhibitor, and licorice was also found to act as serotonin reuptake inhibitor. However, licorice should not be taken by patients with hypertension or heart problems (Janet Contursi, www.livestrong.com, "Natural Herbs As Serotonin Reuptake Inhibitors", April 16, 2011).

EP-A-1634602 describes the use of plants of the genus *Sideritis* for use in treating disorders directed to an altered serotoninergic neurotransmission.

WO-A-2011/076867 describes plant extracts and their use for treating neurodegenerative diseases. The plant extracts used therein are from plants of the family of *Lamioideae.*

M. R. Loizzo et al., Food and Chemical Toxicology 59 (2013) 586-594 describe essential oils of several Cistus species in prevention of neurodegenerative disorders.

The object of the present invention is to provide a composition for use in inhibiting the reuptake of serotonin and/or inhibiting acetylcholinesterase, which can be economically prepared and which causes no side-effects at all or only minor side-effects when administered to a patient.

This object is achieved by a composition for use according to claim 1 and 2. Preferred embodiments are set forth in the subclaims 3 to 6.

### Detailed description of the invention

The extract is selected from *Cistus incanus* and *Cistus salviifolius.*

*Cistus* is a genus belonging to the family of *Cistaceae* covering about 20 species of flowering plants, which are found on dry or rocky soils throughout the Mediterranean region, from Morocco and Portugal through to the Middle East, and also on the Canary Islands.

Plants of the genus *Cistus* contain essential oils containing approximately 60% by weight of diterpenes with manoyl oxide as main component of the diterpenes. They furthermore contain a resin fraction comprising labdane diterpene alcohols and labdane diterpene acids, triterpene acids of the damarane type and fatty acid esters. Further components of *Cistus* are flavonoids, such as apigenin, luteolin, quercetin, myricetin and kaempferol, as well as methylether derivatives and glycosides thereof.

According to the present invention, the term "extract" is used representatively for all products that are obtained from an herbal subject by means of an extraction with a solvent, such as with maceration or percolation.

Generally, an extraction of the plant parts including leaves, twigs, blossoms, buds and fruits with a suitable solvent takes place. Water or a mixture of water with methanol or ethanol is used as a solvent.

The extraction is normally carried out at temperatures of 25 °C to, where applicable, as high as the boiling point of the solvent used. Preferred is an extraction at 95 to 100 °C.

The extraction is normally carried out for 2 to 8 h. Preferably, the extraction is carried out for 3 to 6 h, more preferably for 4 to 5 h.

In order to achieve the highest possible yield, the plant material can be extracted a number of times. Preferably, the extraction is repeated 2 to 6 times, more preferably 3 times.

As a result of the extraction, a liquid, semi-solid or solid raw product is obtained, which can be used in this form for producing a composition for use according to the present invention in inhibiting the reuptake of serotonin or in inhibiting acetylcholinesterase.

A maceration procedure is normally performed for five to nine days, preferably for seven days, at room temperature with a mixture of water and ethanol, by pouring the solvent mixture over the plant elements and letting this stand for the period of time mentioned.

According to the present invention, a percolation of the plant parts is normally achieved by treating the parts with water at 95 to 100 °C for four to five hours by conducting the water through the plant parts.

The crude product obtained from an extraction with a solvent, such as a maceration or percolation, can also be concentrated and/or dried and/or further processed before use. The further processing can, for example, include cleaning steps known to the person skilled in the art, such as centrifugation, filtration and decanting, in order to remove suspended materials from the extract. Chromatography, such as column chromatography, gas chromatography or HPLC or steam distillation may also be used for purification. In a preferred embodiment the crude product is used without further purification steps.

An extract obtained in this way can subsequently be further processed into a dry extract. To produce the dry extract, the solvent can be withdrawn from the liquid raw extract, the concentrated extract or the cleaned extract by, for example, spray drying, freeze drying or vacuum drying.

In a further preferred embodiment in combination with one of the embodiments listed above or below, the composition according to the present invention comprises an extract of at least 2, 3, or 4 plants. More preferably, the composition comprises an extract of two or three plants.

In another preferred embodiment in combination with one of the embodiments listed above or below, the additional plant is selected from the genus *Cistus, Papaver, Chicorium, Elymus,* and *Rubus.* More preferably, the plant is selected from *Papaver rhoeas, Cichorium intybus, Elymus repens,* and *Rubus fruticosus.* Most preferably, the plant is selected from *Cichorium intybus.*

The additional plant may be further selected from the genus *Mentha.* More preferably, the plant is selected from *Mentha aquatica.*

In a further preferred embodiment in combination with one of the embodiments listed above or below, the composition according to the present invention comprises a combination of an extract of *Cistus incanus* and *Cistus salviifolius, Cistus incanus* and *Papaver rhoeas, Cistus incanus* and *Cichorium intybus, Cistus incanus* and *Elymus repens, Cistus incanus* and *Rubus fruticosus, Cistus salviifolius* and *Papaver rhoeas, Cistus salviifolius* and *Cichorium intybus, Cistus salviifolius* and *Elymus repens,* or *Cistus salviifolius* and *Rubus fruticosus.* More preferably, the composition is a combination of an extract of *Cistus incanus* and *Cistus salviifolius, Cistus incanus* and *Papaver rhoeas, Cistus incanus* and *Cichorium intybus, Cistus incanus* and *Elymus repens,* or *Cistus incanus* and *Rubus fruticosus,* most preferably the composition is a combination of an extract of *Cistus incanus* and *Cichorium intybus.*

In a preferred embodiment in combination with any one of the embodiments listed above or below, the composition according to the present invention comprises an extract of a first plant and an extract of another plant in a ratio of 1 : 10 to 10 : 1, preferably 1 : 5 to 5 : 1, and more preferably 1 : 2 to 2 :1, and in particular 1:1.

In another preferred embodiment in combination with one of the embodiments listed above or below, the extract is of the aerial parts of the plant. More preferably, the extract is from the leaves, twigs, blossoms and/or buds.

In the present invention the term "aerial parts of a plant" refers to all parts which are aboveground, including leaves, twigs, blossoms, buds, fruits and seeds. For preparing the extract used according to the present invention, preferably the leaves, twigs, blossoms and buds are used.

In the present invention, the term "twig" refers to a small shoot or branch having a diameter of 3 cm at most. Preferably, the diameter of the twigs is up to 1 cm.

In the present invention, the term "prophylaxis" refers to a procedure to prevent a disease. Prophylactic measures can be divided into primary prophylaxis (prevention of the disease) and secondary prophylaxis (protection from the disease).

In another preferred embodiment in combination with one of the embodiments listed above or below, the composition of the present invention is in liquid, dry or semi-solid form.

In a preferred embodiment in combination with one of the embodiments listed above or below, the composition of the present invention is in the form of a tablet, capsule, powder, tea mixture, granulate, sirup, drops, tincture, emulsion, ointment, cream, gel, paste, infusion, inhalation solution or mouth spray.

The composition according to the present invention can be applied in each of the application forms familiar to the person skilled in the art for both medical and non-medical use. In a preferred embodiment in combination with one of the embodiments listed above or below, the composition is applied in solid form as a tablet, such as a coated tablet, effervescent tablet, sugar-coated tablet, chewing tablet, lozenge, pill, chewing gum, capsule, powder, tea mixture, or granulate. In an alternative preferred embodiment in combination with one of the embodiments listed above or below, the composition according to the present invention is applied in liquid form as mouthwash, sirup, drops, tincture, emulsion, infusion, inhalation solution or gargling solution. In another preferred embodiment in combination with one of the embodiments listed above or below, the composition according to the present invention is applied in a semi-solid form as ointment, cream, gel, or paste. In a further preferred embodiment in combination with one of the embodiments listed above or below, the composition according to the present invention is applied as a spray, such as a mouth spray. In particular, the composition is applied as a tablet, sirup, tincture, tea mixture, and infusion.

In galenic and other application forms, the composition of the present invention can be processed with the customary galenic aids, such as tablet bonders, filling agents, preservative agents, tablet-opening agents, flow regulation agents, softening agents, wetting agents, dispersing agents, emulsifying agents, solvents, retarding agents, anti-oxidative agents, consistency regulators, penetration improvers and/or propellant gases. Further elements, such as vitamins and minerals, can be added to the composition according to the present invention. The concentration of the composition in the application form varies, depending on the type of application. As a rule, the quantity of the composition of the present invention amounts to between 0.5 and 1,000 mg per dosing unit for solid application forms. Preferably the quantity of the amounts to between 1 and 500 mg per unit. In liquid application forms, the composition can be in a concentration of 0.1 µg/mL to 500 mg/ml, preferably from 1 pg/mL to 200 mg/mL, more preferably from 1 to 100 mg/mL, in particular 5 to 50 mg/mL. In the case of semi-solid application forms, the content of the composition amounts to 1 to 90% by weight, preferably 5 to 75 % by weight, based on the total weight of the application form.

The following examples further explain the present invention.

### Examples

### General procedure for the production of liquid and dry extracts:

The collected plant material is visually checked, and non-original, damaged or eaten away parts are removed.

The purified material is spread on a table in a green house and covered with paper for drying. The plant parts are turned around on a daily basis and visually checked for non-original and damaged parts, which are removed. The residual moisture of the plant material is determined on regular basis. The material is good for further processing when the residual water content is at a maximum of 10% by weight. Alternatively, the purified material is dried with a belt dryer according to standard procedures known to the person skilled in the art.

The plant material is coarsely cut and transferred to a beaker; Cold, distilled water is added (10 to 30-times the quantity of the plant material). The resulting mixture is heated on a hotplate while being stirred until it starts boiling. Simmering is continued for 1 h.

The hot liquid is strained, and the residual plant material is squeezed out. The resulting aqueous extract is filled directly in a bottle.

For the production of the dry extract, the liquid extract is filled in metal bowls and concentrated at 80 °C in a drying oven until the solvent has completely evaporated. The residue is scraped out of the metal bowl and weighed. Alternatively, the liquid extract is freeze-dried according to standard procedures known to the person skilled in the art.

### Synaptosome preparation

Male Sprague Dawley rats were sacrificed by decapitation, various regions of rat brain (striatum, cortex and hypothalamus) were dissected on ice. The tissues were homogenized in 20 volumes of ice-cold sucrose (0.32 M) and centrifuged for 10 min at 1000 x g at 4 °C. Resulting supernatants with crude synaptosomes were stored on ice until usage.

### Serotonin uptake assay

The serotonin uptake assay was performed using a similar protocol as Perovic and Müller (1995) used for serotonin uptake into synaptosomes (Perovic, S.; Müller, W. E., Pharmacological profile of hypericum extract. Effect on serotonin uptake by postsynaptic receptors, Arzneimittelforschung 1995, 45, 1145-1148).

| ligand | concentration | non specific | incubation | detection method |
|---|---|---|---|---|
| [³H]-Serotonin | 200000 dpm | Imipramine hydrochlorid (31.6 µM) | 20 min, 37 °C, in the dark | scintillation counting |

The incubation buffer contained 118 mM NaCl, 11 mM glucose, 1.2 mM NaH₂PO₄, 5 mM KCI, 25 mM NaHCO₃, 50 µM ascorbic acid., 10 µM EGTA and 2.5 mM MgSO₄; pH 7.4.

The assay was initiated by addition of [³H]-serotonin to rat synaptosomes (from cortex) and graded concentrations of test compound in incubation buffer. After incubation for 20 min at 37 °C in the dark the assay was terminated by addition of 1.5 mL ice cold incubation buffer and rapid transfer of the samples on GF/C filter plates (PerkinElmer), presoaked in incubation buffer and filter-bound radioactivity was determined by a microplate reader (Microbeta, Wallac, Finnland).

### Dopamine uptake assay (Reference)

The dopamine uptake assay was performed using a similar protocol as Perovic and Müller (1995) used for serotonin uptake into synaptosomes.

| ligand | concentration | non specific | incubation | detection method |
|---|---|---|---|---|
| [³H]-Dopamine | 100000 dpm | GBR 12909 (3.16 µM) | 15 min, 37 °C, in the dark | scintillation counting |

The incubation buffer contained 118 mM NaCl, 11 mM glucose, 1.2 mM NaH₂PO₄, 5 mM KCI, 25 mM NaHCO₃, 200 µM ascorbic acid., 10 µM EGTA , 2.5 mM MgSO₄ and 10 µM Pargylin; pH 7.4.

The assay was initiated by addition of [³H]-dopamine to rat synaptosomes (from striatum) and graded concentrations of test compound in incubation buffer. After incubation for 15 min at 37°C the assay was terminated by addition of 1.5 mL ice cold incubation buffer and rapid transfer of the samples on GF/C filter plates (PerkinElmer), presoaked in incubation buffer and filter-bound radioactivity was determined by a microplate reader (Microbeta, Wallac, Finnland).

### Norepinephrine uptake assay (Reference)

The norepinephrine uptake assay was performed using a similar protocol as Perovic and Müller (1995) used for serotonin uptake into synaptosomes.

| ligand | concentration | non specific | incubation | detection method |
|---|---|---|---|---|
| [³H]-Norepinephrine | 100000 dpm | Protriptyline (1µM) | 15 min, 37 °C, in the dark | scintillation counting |

The incubation buffer contained 118 mM NaCl, 11 mM glucose, 1.2 mM NaH₂PO₄, 5 mM KCl, 25 mM NaHCO₃, 200 µM ascorbic acid., 10 µM EGTA , 2,5 mM MgSO₄ and 10 µM Pargylin; pH 7.4.

The assay was initiated by addition of [³H]-norepinephrine to rat synaptosomes (from cortex or hypothalamus) and graded concentrations of test compound in incubation buffer. After incubation for 15 min at 37°C the assay was terminated by addition of 1.5 mL ice cold incubation buffer, washed with incubation buffer and rapid transfer of the samples on GF/C filter plates (PerkinElmer), presoaked in incubation buffer and filter-bound radioactivity was determined by a microplate reader (Microbeta, Wallac, Finnland).

### Acetylcholinesterase enzyme assay

The Amplite colorimetic acetylcholinesterase assay kit uses DTNB to quantify the thiocholine produced from the hydrolysis of acetylthiocholine by acetylcholinesterase. The absorption intensity of DTNB adduct is used to measure the amount of thiocholine formed, which is proportional to the acetylcholinesterase activity.

The enzyme assay was performed in principle as suggested by the supplier of the kit with the exception that a fixed concentration of enzyme was used and serial dilutions of the reference inhibitor neostigmine or the test compounds.

The signal was read after 30 min incubation at room temperature by an absorbance microplate reader (Sunrise, Tecan Deutschland GmbH, Crailsheim, Germany) at 405 nm.

### Data analysis and expression of results (uptake)

The data of the reference compound (subsection: assay validation) is presented as total membrane-bound radioactivity (in cpm).

Test compound data are presented as percent control uptake: 100% control uptake corresponds to the uptake in the absence of any test compounds; 0% control serotonin, dopamine or norepinephrine uptake corresponds to the filter-bound radioactivity in the presence of 10 µM Imipramine (serotonin uptake), 3.16 µM GBR 12909 (dopamine uptake) or 1 µM protriptyline (norepinephrine uptake).

The IC₅₀ values (concentration causing half-maximal inhibition of control uptake were determined by non-linear regression analysis of the competition curves using the algorithm "sigmoidal dose-response" (GraphPadPrism, San Diego, USA). In case of ill-defined curves or algorithm-generated minima below the 0 % control uptake the IC₅₀ value was determined by graphical extrapolation.

### Data analysis and expression of results (acetylcholinesterase)

The data of the reference compound (subsection: assay validation) is presented in absorption units at 405 nm.

Test compound data are presented as percent control enzyme activity: 100% control enzyme activity corresponds to the enzyme activity in the absence of any inhibitor or test compound; 0% enzyme activity corresponds to the maximal inhibition by the reference compound neostigmine. In case of colored test compounds which display an absorption at 405 nm and therefore interfere with the assay from every data point the absorption at 405 nm in the absence of the enzyme preparation was subtracted.

The IC₅₀ values (concentration causing half-maximal inhibition of control enzyme activity) was determined by non-linear regression analysis of the curves using the algorithm "sigmoidal dose-response" (GraphPadPrism, San Diego, USA). In case of ill-defined curves or algorithm-generated minima below 0 % control activity the IC₅₀ value was determined by graphical extrapolation.

### Results

### Serotonin uptake assay

### Sample preparation:

Samples of *Papaver rhoeas, Cistus incanus, Elymus repens, Rubus fructosus* and *Cichorium intybus* were prepared as liquid extracts according to the above described General Procedure in the given concentration.

Samples of *Cistus salviifolius* and the mixture of *Cistus incanus* and *Cichorium intybus* were prepared from the dry extracts. The dry extracts were prepared according to the above described General Procedure. The dry extracts of *Cistus salviifolius* and a mixture (1 : 1) of *Cistus incanus* and *Cichorium intybus* were solubilized in H₂O/DMSO (50% by volume DMSO) at a concentration of 10 mg/mL and sonified for 5 to 15 minutes.

| Extract Concentration IC₅₀ [µg/mL] [g/100 mL] (1. Measurement) | | | IC₅₀ [µg/mL] (2. Measurement) | IC₅₀ [µg/mL] mean |
|---|---|---|---|---|
| *Papaver rhoeas* | 1.37 | 9.8 | 9.8 | 9.8 |
| *Cistus incanus* | 0.58 | 32 | 44 | 38 |
| *Elymus repens* | 0.52 | 23 | 25 | 24 |
| *Cistus salviifolius* | 1.00 | 105 | 115 | 110 |
| *Rubus fructosus* | 0.49 | 88 | 119 | 104 |
| *Cichorium intybus* | 0.45 | 30 | 33 | 32 |
| *Cistus incanus + Cichorium intybus* | 1.00 (mixt. 1 : 1) | 91 | 135 | 113 |

### Dopamin uptake assay (Reference)

### Sample preparation:

Samples of *Cistus incanus, Cistus salviifolius, Cichorium intybus* and the mixture of *Cistus incanus* and *Cichorium intybus* were prepared from the dry extracts. The dry extracts were prepared according to the above described General Procedure. The dry extracts of *Cistus incanus, Cistus salviifolius, Cichorium intybus* and a mixture (1 : 1) of *Cistus incanus* and *Cichorium intybus* were solubilized in H₂O/DMSO (50% by volume DMSO) at a concentration of 10 mg/mL and sonified for 5 to 15 minutes.

| Extract | Concentration [g/100 mL] | IC₅₀ [µg/mL] (1. Measurement) | IC₅₀ [µg/mL] (2. Measurement) | IC₅₀ [µg/mL] mean |
|---|---|---|---|---|
| *Cistus incanus* | 1.00 | 32 | 23 | 28 |
| *Cistus salviifolius* | 1.00 | 33 | 40 | 37 |
| *Cichorium intybus* | 1.00 | 49 | 105 | 77 |
| *Cistus incanus + Cichorium intybus* | 1.00 (mixt. 1 : 1) | 63 | 54 | 59 |

### Norepinephrine uptake assay (Reference)

### Sample preparation:

Samples of *Cistus incanus, Cistus salviifolius, Cichorium intybus,* the mixture of *Cistus incanus, Cichorium intybus* and *Papaver rhoeas* were prepared from the dry extracts. The dry extracts were prepared according to the above described General Procedure. The dry extracts of *Cistus incanus, Cistus salviifolius, Cichorium intybus* and a mixture (1 : 1) of *Cistus incanus* and *Cichorium intybus* were solubilized in H₂O/DMSO (50% by volume DMSO) at a concentration of 10 mg/mL and sonified for 5 to 15 minutes. The dry extract of *Papaver rhoeas* was dissolved in pure water at a concentration of 10 mg/mL.

| Extract | Concentration [g/100 mL] | IC₅₀ [µg/mL] (1. Measurement) | IC₅₀ [µg/mL] (2. Measurement | IC₅₀ [µg/mL] mean |
|---|---|---|---|---|
| *Cistus incanus* | 1.00 | 27 | 13 | 20 |
| *Cistus salviifolius* | 1.00 | 19 | 20 | 20 |
| *Cichorium intybus* | 1.00 | 43 | 71 | 57 |
| *Cistus incanus* + *Cichorium intybus* | 1.00 (mixt. 1 : 1) | 28 | 29 | 29 |
| *Papaver rhoeas* | 1.00 | 110 | 72 | 91 |

### Inhibition of acetylcholinesterase

### Sample preparation:

A sample of *Cistus incanus* was prepared as liquid extracts according to the above described General Procedure in the given concentration.

A sample of *Cistus salviifolius* was prepared from a dry extract. The dry extract was prepared according to the above described General Procedure. The dry extract of *Cistus salviifolius* was solubilized in H₂O/DMSO (50% by volume DMSO) at a concentration of 10 mg/mL and sonified for 5 to 15 minutes.

| Extract | Concentration [g/100 mL] | IC₅₀ [µg/mL] (1. Measurement) | IC₅₀ [µg/mL] (2. Measurement) | IC₅₀ [µg/mL] mean |
|---|---|---|---|---|
| *Cistus incanus* | 0.46 | 20 | 27 | 24 |
| *Cistus salviifolius* | 1.00 | 48 | 56 | 52 |

As can be seen from the examples, the compositions in accordance with the present invention are able to inhibit the reuptake of serotonin and to inhibit acetylcholinesterase.

### Electropharmacogram Screening

### Introduction:

Drugs exert their action within the organism by interaction with targets defined biochemically (receptors, enzymes, channels transporters et cetera), large protein molecules sometimes also sitting at the outer surface of cells. With respect to the central nervous system neurotransmitter receptors represent main targets of drugs. Interaction of drugs with these molecules induces a signaling cascade, which finally ends up with the control of ion channel conductance. Since the electric activity of single neurons depends on the set of momentarily active ion channels, communication between neurons is governed by channel activity. From here, it is obvious that field potentials contain the information of larger local networks of electrically active neurons, by reflecting the interaction of drugs with their targets within the concert of neurotransmission. Frequency analysis of the field potentials in the presence of drugs leads to the so called electropharmacogram (Dimpfel, W., Br. J. Pharmacol. 2007, 152, 538-548). Interpretation of the results was performed with respect to neurotransmitter activity as well as aiming at possible clinical indications in humans. A relationship between EEG delta waves and cholinergic neurotransmission has been suggested by Dimpfel (Eur. Neuropsychopharmacol. 2005, 15, 673-682). Theta waves have been recognized as being influenced by drugs acting at the norepinephrine alpha2 receptor (Dimpfel, W., Schober, F., Brain Pharmacology 2001, 1, 89-97). Presynaptic interaction with this receptor leads to drowsiness and sleep and changes of theta waves have been used as part of a formula describing depth of sleep in humans (Dimpfel et al., Eur. J. Med. Res. 1998, 14, 453-460). Dopaminergic activity is reflected by changes in alpha2 frequencies (Dimpfel, W., Neuropsychobiology 2009, 58, 178-186).

### General:

Quantitative field potential analysis from freely moving adult day-night converted rats was used to obtain spectral signatures of electric brain activity with respect to four deep brain areas, namely frontal cortex, hippocampus, striatum and reticular formation. Experiments were conducted in a crossover design with 6 rats carrying an implant of 4 stainless steel electrodes. Data were transmitted wirelessly and analyzed by Fast Fourier Transformation. After a pre-drug phase of 45 minutes extracts were administered by a gavage in 1 ml vehicle. Recording was maintained for another 5 hours.

### Experimental Procedure:

EEG signals were recorded from frontal cortex, hippocampus, striatum and midbrain reticular formation of freely moving rats from inside a totally copper-shielded room. Signals were wirelessly transmitted by a radio-telemetric system (Rhema Labortechnik, Hofheim, Germany, using 40 Megahertz as carrier frequency) and were amplified and processed to give power spectra of 0.25 Hz resolution (Dimpfel et al., Neuropsychobiology 1986, 16, 163-168; Dimpfel et al., Neurobiology 1988, 19, 116-120; Dimpfel et al. Arzneimittelforschung/Drug Research 1989, 39, 560-563; Dimpfel, W., Eur. J. Med. Res. 2003, 8, 199-207). After automatic artefact rejection signals were collected in sweeps of 4 s duration and Fast Fourier transformed using a Hanning window. Sampling frequency was 512 Hz. Four values were averaged to give a final sampling frequency of 128 Hz, well above the Nyquist frequency. The resulting electrical power spectra were divided into 6 specially defined frequency ranges (delta: 0.80 - 4.50 Hz; theta: 4.75 - 6.75 Hz; alphal: 7.00 - 9.50 Hz; alpha2: 9.75 - 12.50 Hz; beta1: 12.75 -18.50 Hz; beta2: 18.75 - 35.00 Hz). Spectra were averaged in steps of 3 minutes each and displayed on-line. In an offline procedure spectra were averaged to give longer periods for further analysis and data presentation.

### Test Items:

Preparations were tested by oral administration. The "Tele-Stereo-EEG" animal model consisting of continuous recording of intracerebral field potentials was used in combination with a video tracking system for detection of changes in motility (GJB Datentechnik GmbH, D-98704 Langewiesen, Germany). This system recognized locomotion as well as stereotyped behaviour by following a contrast difference of the black transmitter on the head of the animal in comparison to its environment. The system has been validated in a previous study with different dosages of caffeine.

Solutions were prepared freshly for each experimental day and administered orally after 45 minutes of pre-drug baseline recording.

Samples of *Cistus incanus, Cichorium intybus, Mentha aquatica and Elymus repens* were prepared as dry extracts according to the above described General Procedure. 50.0 g of coarsely cut *Cistus incanus* are heated in 1500 mL of distilled water for 1 h. The resulting aqueous extract is dried according to standard procedures known to the person skilled in the art. 50.0 g of coarsely cut *Cichorium intybus* are heated in 1500 mL of distilled water for 1 h. The resulting aqueous extract is dried according to standard procedures known to the person skilled in the art. 50.0 g of coarsely cut *Mentha aquatica* are heated in 1500 mL of distilled water for 1 h. The resulting aqueous extract is dried according to standard procedures known to the person skilled in the art. 50.0 g of coarsely cut *Elymus repens* are heated in 1500 mL of distilled water for 1 h. The resulting aqueous extract is dried according to standard procedures known to the person skilled in the art.

### Animals and Management

Six adult Fisher 344 rats (8 month of age and day - night converted, weight about 400 g, provided by Charles River Laboratories, D-97633, Sulzfeld) were used in this experimental series. Animals were implanted with electrodes into the brain and were given two weeks for recovery from surgery. After this, the transmitter was plugged in for adaptation and control experiments. During the recording rats were not restricted and could move freely but did not have food available (chewing would have produced too many artefacts). The principles of laboratory animal care were followed in all trials and the local authorities responsible for animal care allowed the performance according to German Health Guidelines.

The animals had been allowed to acclimatize for at least 4 weeks before use. There was automatic control of light cycle, temperature and humidity. Light hours were 18 h in the evening - 6 h in the morning. Daily monitoring indicated that temperature and humidity remained within the target ranges of 22°C +- 2°C and 44% +- 5% respectively. Cages, bedding, and water bottles were changed at regular intervals, i.e. every 2-3 days. Standard Diet (Nohrlin H10, Altromin, D-32791 Lage, Germany) was available to the animals ad libitum. The animals had access to domestic quality mains water ad libitum.

### Surgery:

Rats had been implanted with 4 bipolar concentric steel electrodes within a stereotactic surgical procedure during anaesthesia with Ketamine. All four electrodes were placed 3 mm lateral within the left hemisphere. Dorsoventral coordinates were 4, 6, 4.2 and 8 mm and anterior coordinates were 3.7, 9.7, 5.7 and 12.2 mm for frontal cortex, striatum, hippocampus, and reticular formation, respectively (according to the atlas of Paxinos and Watson, 1982). A pre-constructed base plate carrying 4 bipolar stainless steel semi-micro electrodes (neurological electrodes "SNF 100" from Rhodes Medical Instruments, Inc., Summerland, CA 93067, USA) and a 5-pin-plug was fixed to the skull by dental cement interacting with 3 steel screws placed on distance into the bone. The distant recording spot of the electrode was the active electrode whereas the proximal spots of the four electrodes were connected to each other to give a shortcut common reference. The base plate was carrying a plug to receive later on the transmitter (weight: 5.2 g including battery, 26x12x6 mm of size).

### Treatment:

### Treatment Groups

A crossover design with at least 3 days of drug holidays in between the administrations was used. Controls consisted of oral administration of 1 ml/kg 1% Methylcellulose or saline (0.9% NaCl). After a pre-drug period of 45 minutes for baseline recording, drug effects were observed continuously for 300 minutes subdivided into 60 min periods, after a lag time of 5 minutes for calming of animals after oral administration by gavage. Changes of electrical power (µV² ) are expressed as % of the 45 min. absolute pre-drug spectral power values within each frequency band. Data were averaged from 5-6 animals. Data are expressed as mean values ± S.E.M.

### Selection of Dose Levels

Dose levels were chosen arbitrarily on the base of in vitro data. They will give about the human equivalent dosage according to body surface area (BSA) normalization method (Shannon et al., The FASEB Journal, 2007, 22, 1-3) i.e. rats having about 450 g body weight receive the ten-fold dosage on a kg base in comparison to humans. According to this formula, a dose of 360 to 720 mg extract in humans corresponds to about 45.0 mg/kg in rats.

### Route and Means of Administration

The animals were dosed orally by gavage at a constant dose volume of 1 ml dosing solution per kg of body weight. The volume administered to each animal was determined each day by the weight of that animal at the time of administration.

### Statistical Analysis:

Wilcoxon, Mann, Whitney U-test was used throughout all experimental data for comparison to results obtained by vehicle injection.

### Results:

### Effects of control (Vehicle 1) (1% Methylcellulose)

The electropharmacogram was recorded continuously for 5 h after administration of 1% Methylcellulose (vehicle 1 for control, 1 ml/kg body weight). Minor changes of spectral power within the four brain regions following administration were only observed during the first hour after administration. The pattern remained stable of the next four hours.

### Effects of control (Vehicle 2) (0.9% NaCl)

Changes of spectral power within the four brain regions following administration could not be detected. The pattern remained stable throughout the experimental time of 5 hours.

### Effects of extract of Cistus incanus (45.0 mg/kg)

The extract of *Cistus incanus* was suspended in 1% methylcellulose and administered using a dosage of 45.0 mg/kg. The time course of spectral power in % of the pre-drug values is depicted in Table 1. During the first hour after administration a general attenuation of spectral power emerged in the frontal cortex and striatum. Attenuation of alpha1 and beta1 as well as beta2 power was statistically significant with p<0.05 in the striatum. During the next hour attenuation of alpha1 and beta power persisted, but only attenuation of beta power in the striatum remained statistically significant different from control, despite the fact that attenuation of alpha1 activity was more pronounced within the frontal cortex. The slightly different pattern during the first hour might very well derive from the effects of methylcellulose inducing some attenuation of delta, theta and alpha2 spectral power. Practically, no changes of spectral power were observed in the reticular formation. Thus, the characteristic pattern of spectral changes for the extract of *Cistus incanus* is seen during the second hour after administration.

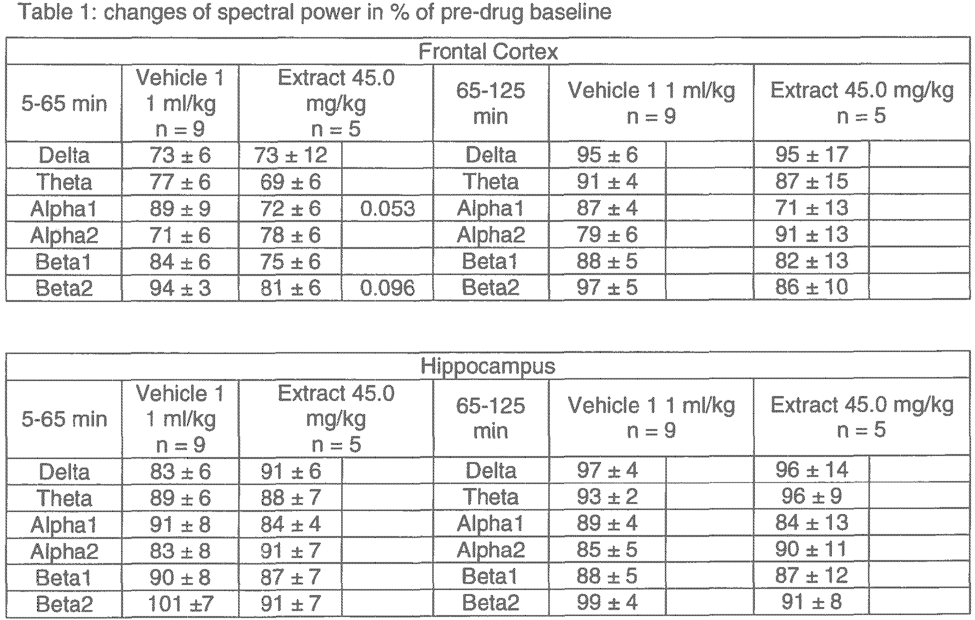

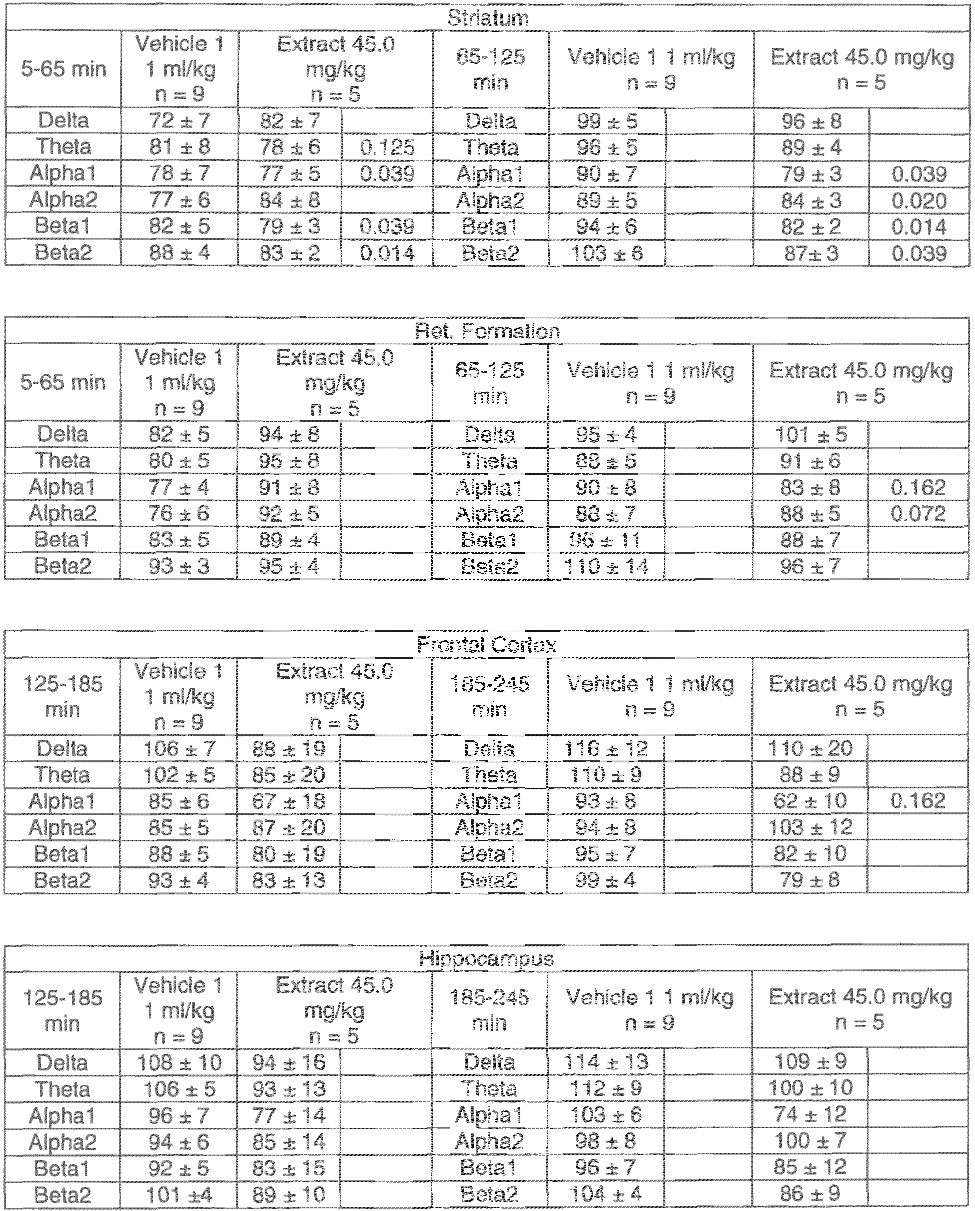

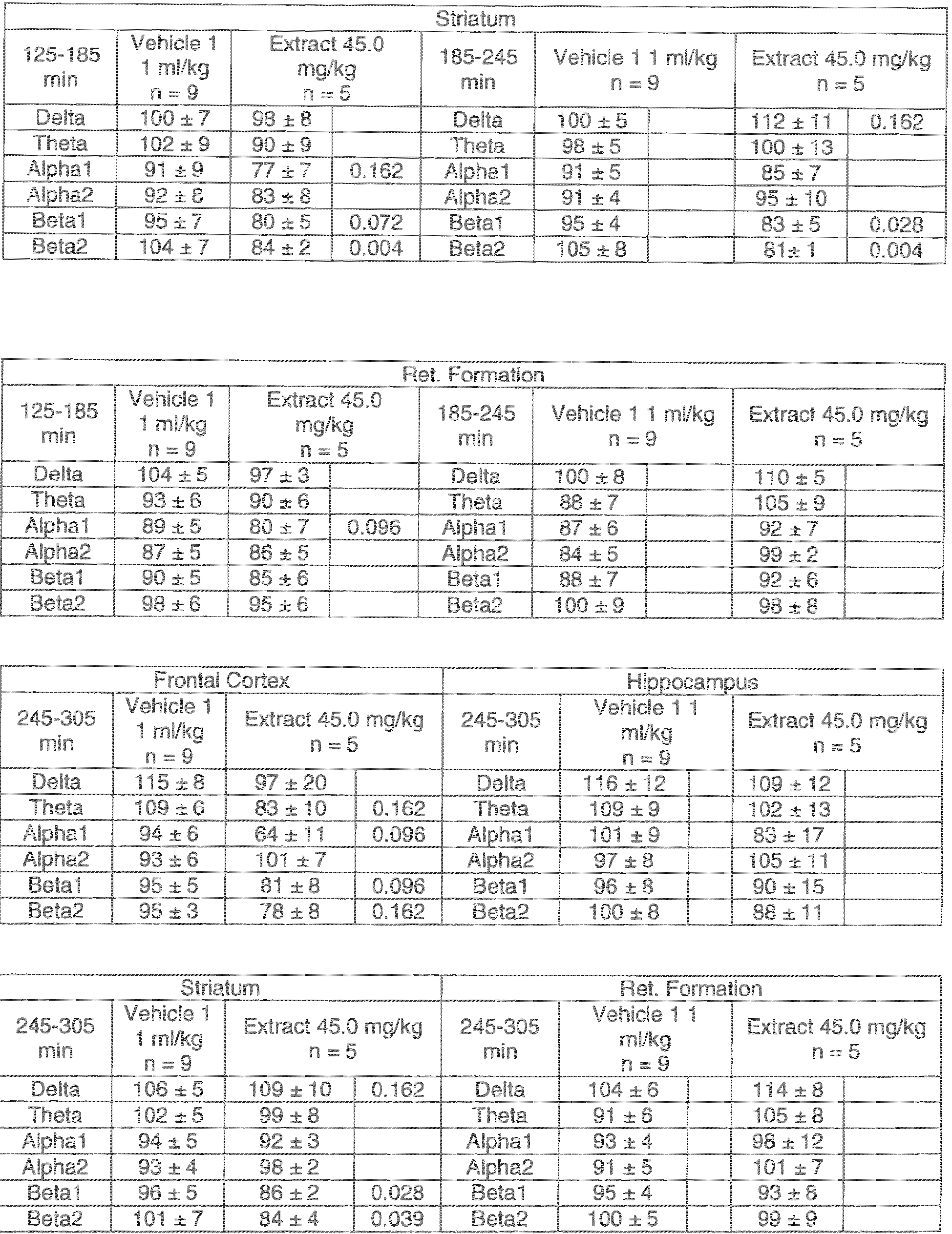

### Effects of extract of Cichorium intybus (45.0 mg/kg) (Reference)

The extract of *Cichorium intybus* was dissolved in physiological saline. Spectral changes of the extract of *Cichorium intybus* were dominated by attenuation of alpha2 power within all brain areas during the first hour after administration. In the hippocampus and reticular formation these changes were statistically significant from control (0.9% NaCl). During the second hour after administration changes persisted with statistical significances in the hippocampus with respect to theta, alpha1, alpha2 and beta1 power. Significant attenuation of alpha2 spectral power lasted into the third hour after administration. In general, attenuation of alpha2 and beta1 power was the prominent features induced by the extract of *Cichorium intybus,* which were visible until the last hour after administration but without statistical significance during the 4th and 5th hour.

The complete time course of spectral changes is depicted in Table 2.

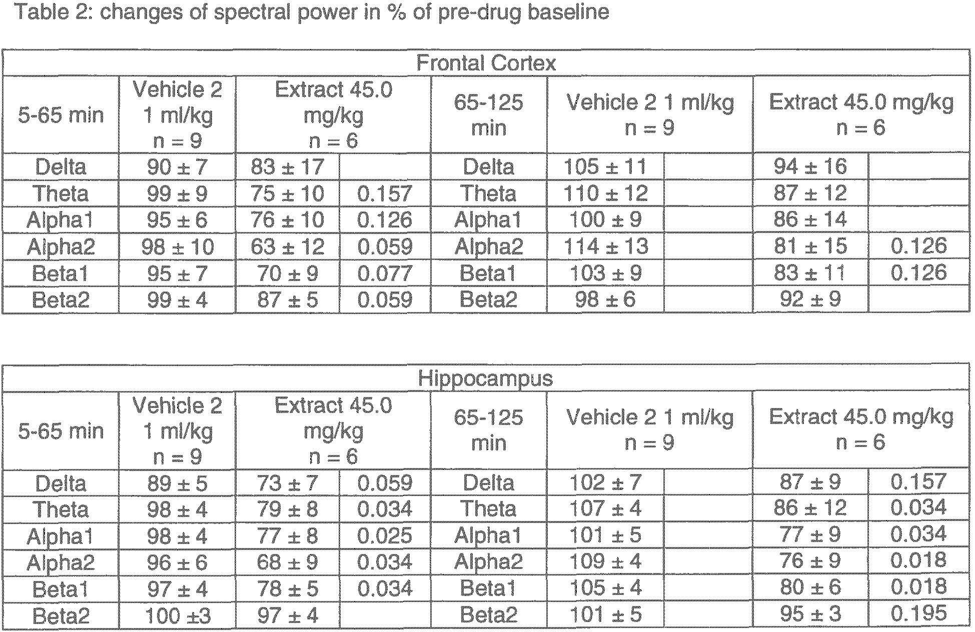

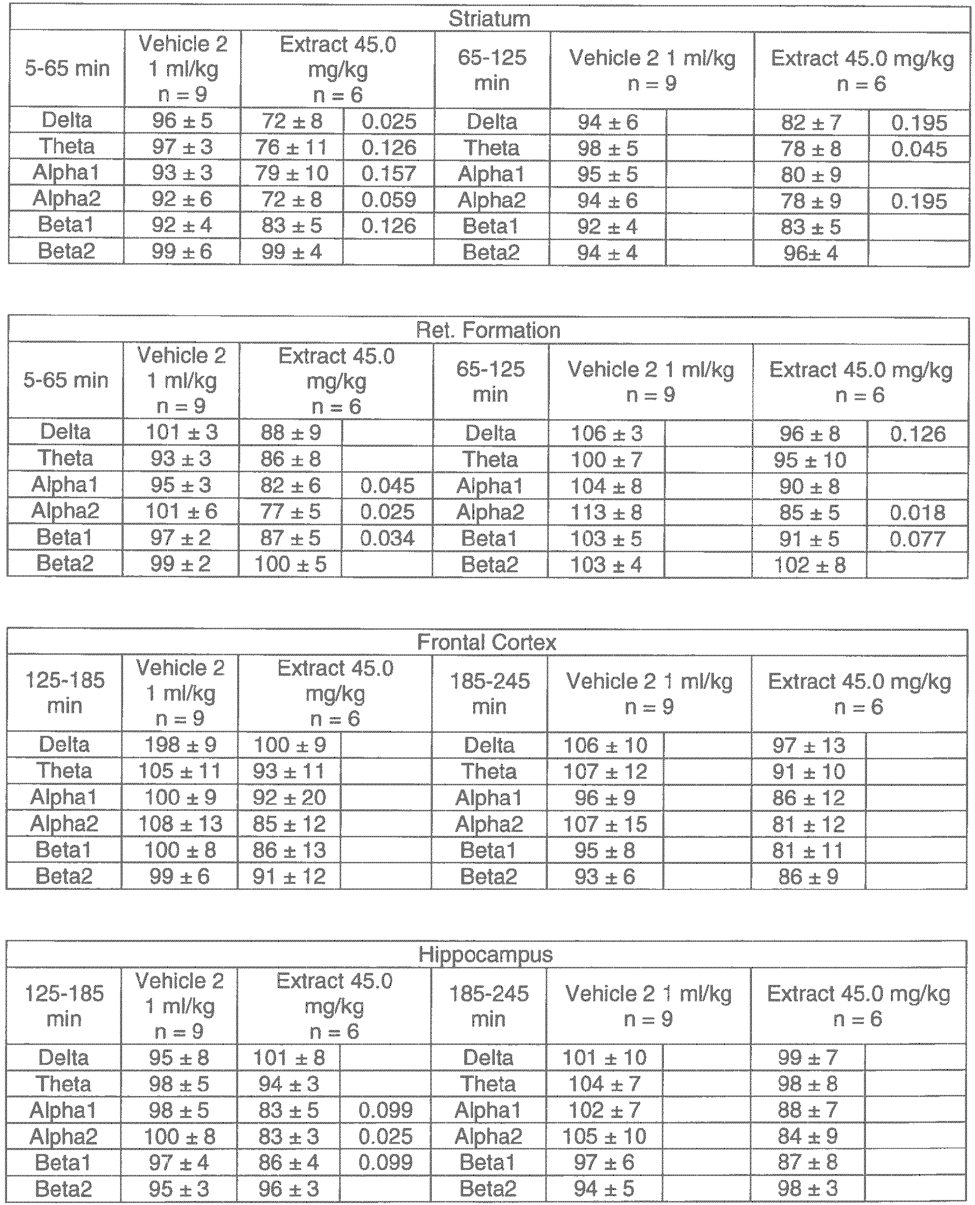

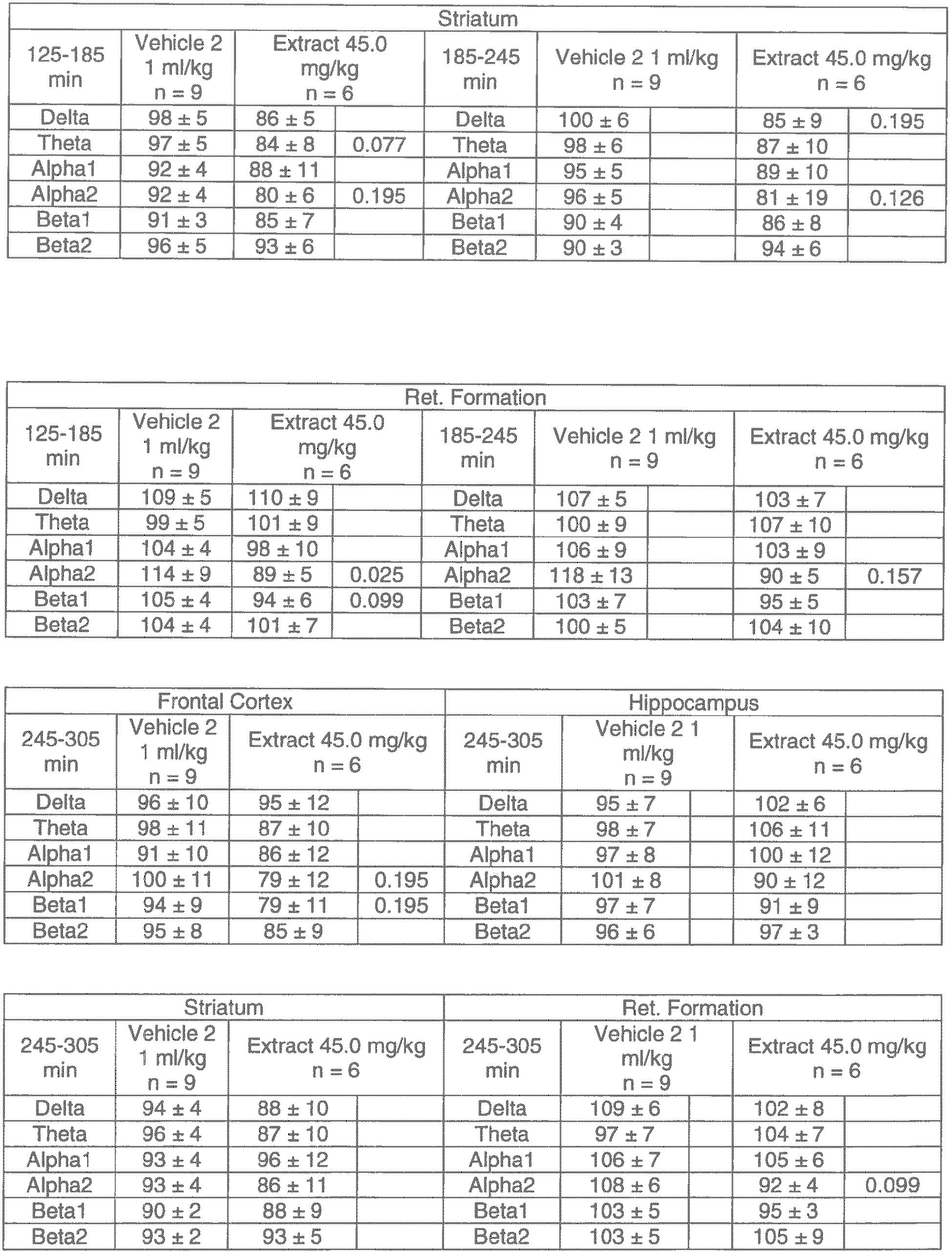

### Effects of extract of Mentha aquatica (100.0 mg/kg) (Reference)

The extract of *Mentha aquatic* was dissolved in saline. A pilot trial with administration of 45.0 mg/kg in 3 animals did not reveal consistent changes of spectral power. Therefore, a dosage of 100.0 mg/kg was prepared. This dosage resulted in attenuation of delta, theta, alpha2 and beta1 spectral power (statistically significant except for beta1 power) with the frontal cortex. During the second hour after administration also statistically significant changes were observed in the hippocampus. Attenuation of alpha1, alpha2 and beta1 power lasted all over the rest of the experimental period within the frontal cortex and hippocampus, but without statistical significance. No consistent changes of the spectral pattern were observed in the reticular formation, similar to the effects of the extract of *Cistus incanus.* The difference to the extract of *Cistus incanus* consists in the additional attenuation of alpha2 power. A time course of the induced changes is documented in Table 3.

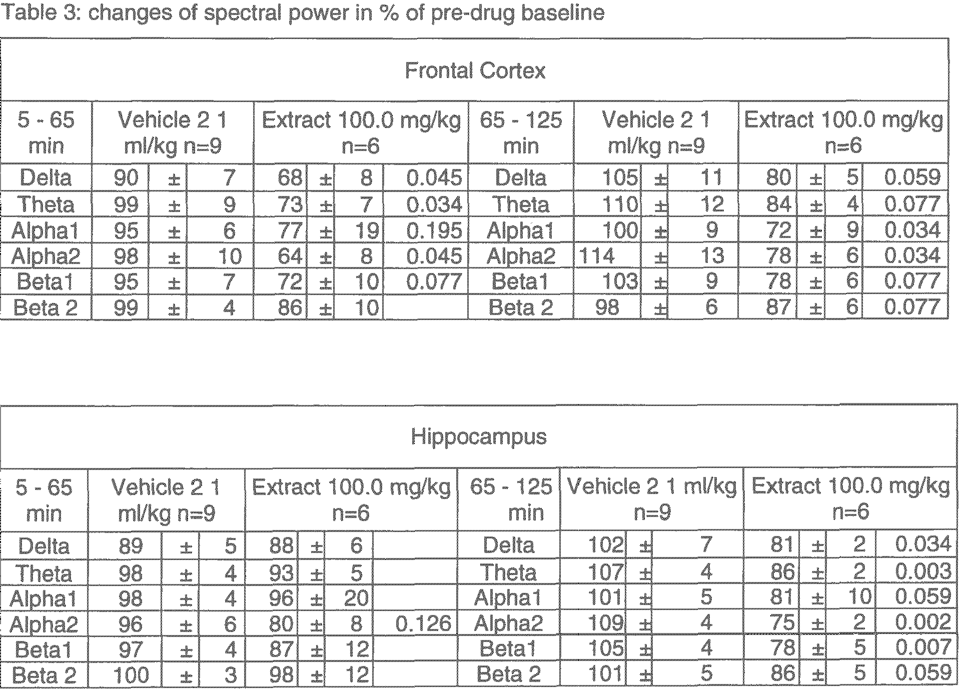

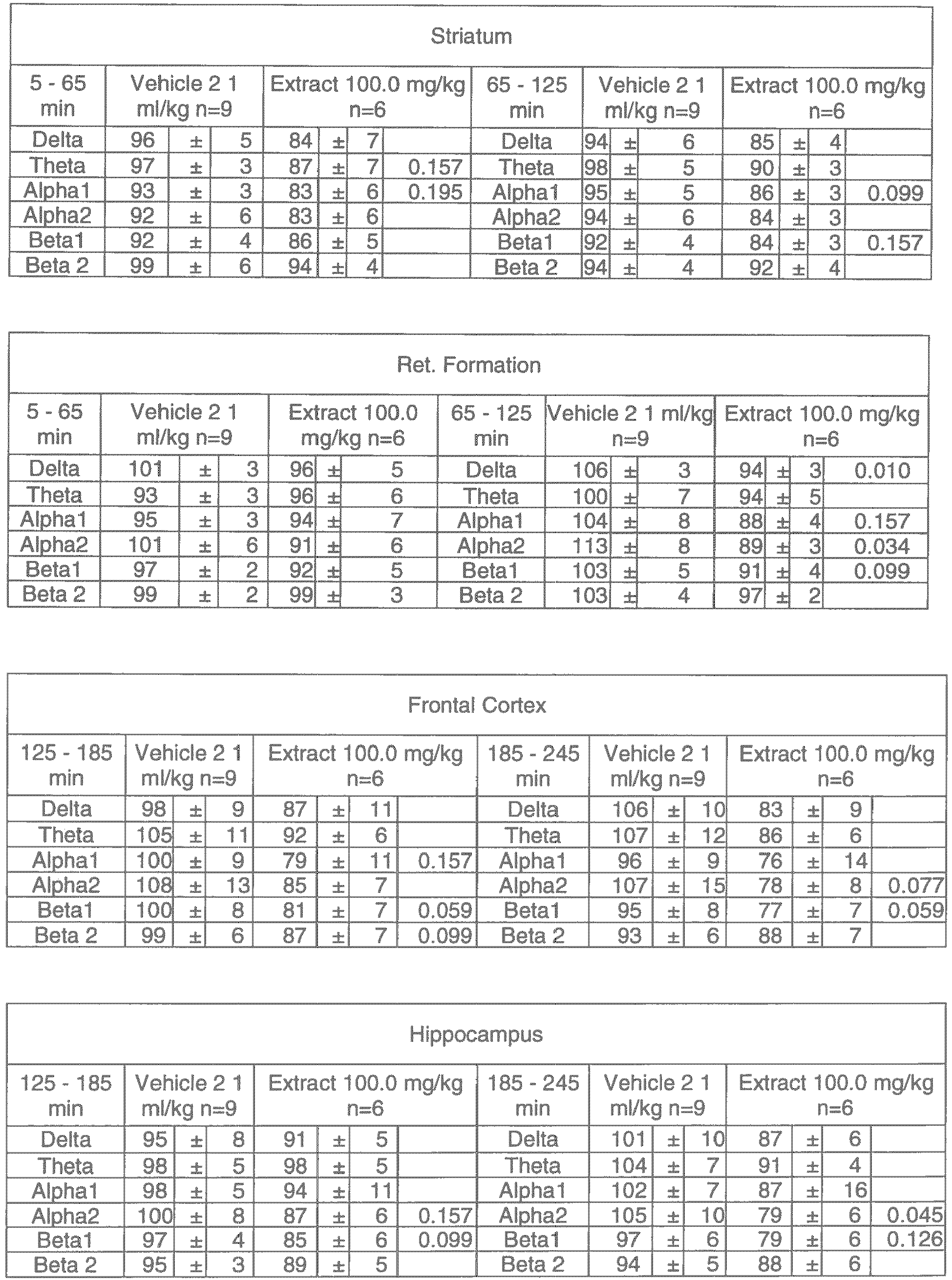

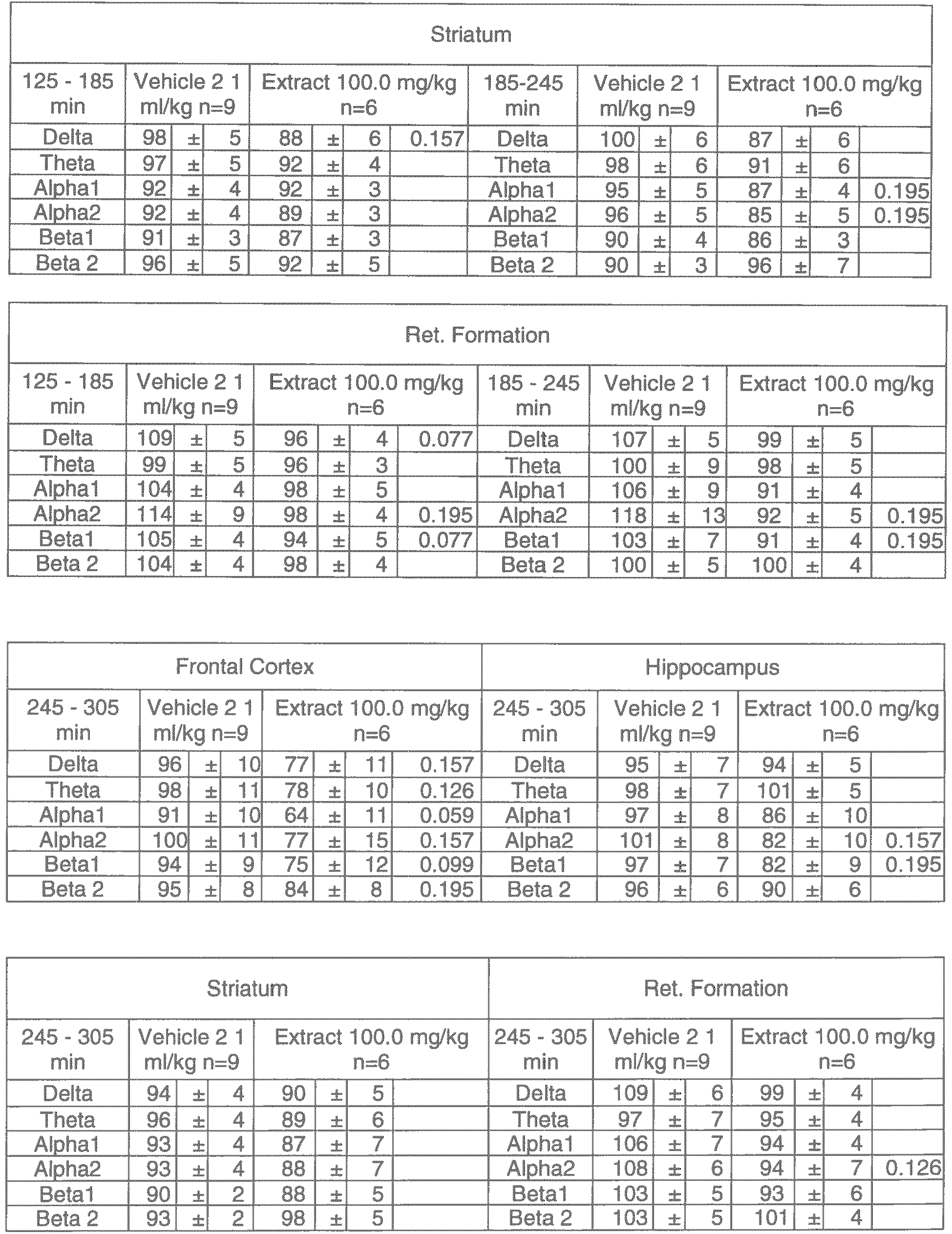

### Effects of extract of Elymus repens (45.0 mg/kg) (Reference)

The extract of *Elymus repens* was also completely dissolved in physiological saline. During the first hour after administration significant attenuation of alpha2 spectral power emerged within all four brain areas lasting into the second hour after administration. In addition, also theta (cortex and striatum) and alpha1 spectral power (hippocampus and reticular formation) was attenuated in a statistically significant manner in comparison to control (saline). The complete time course of spectral changes is documented in Table 4.

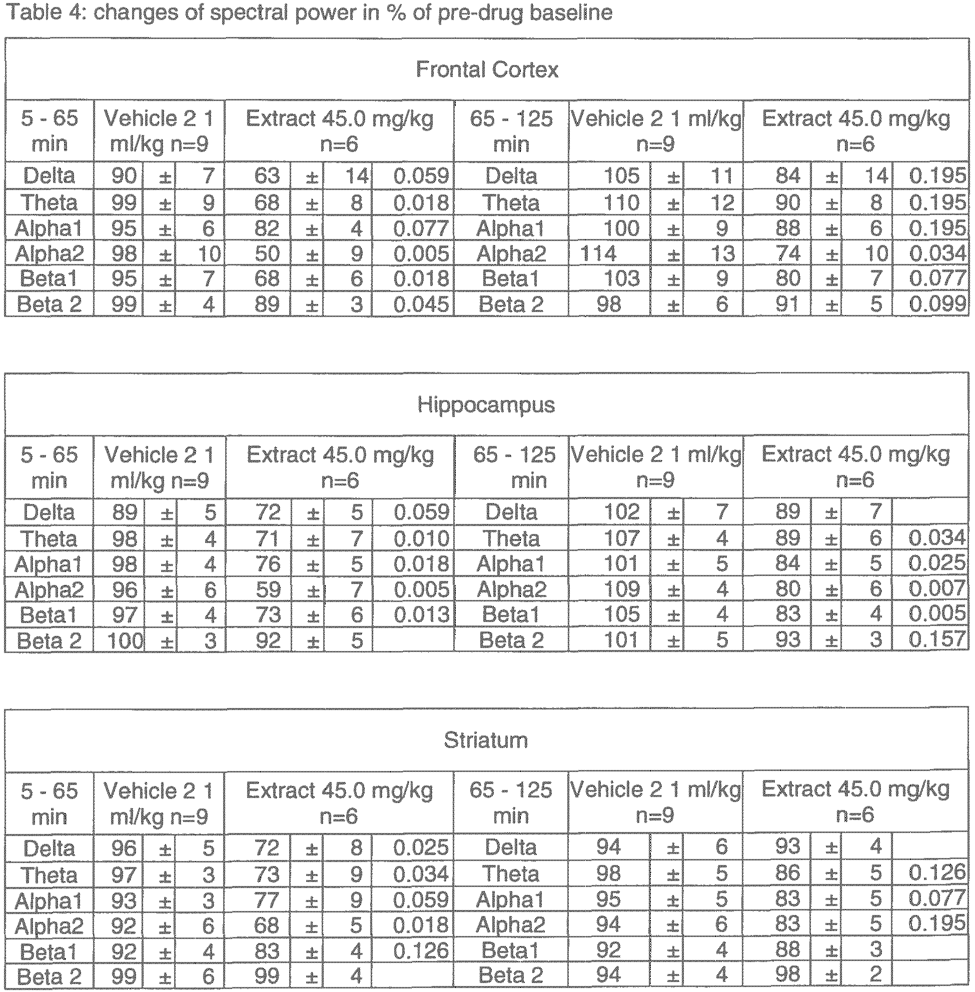

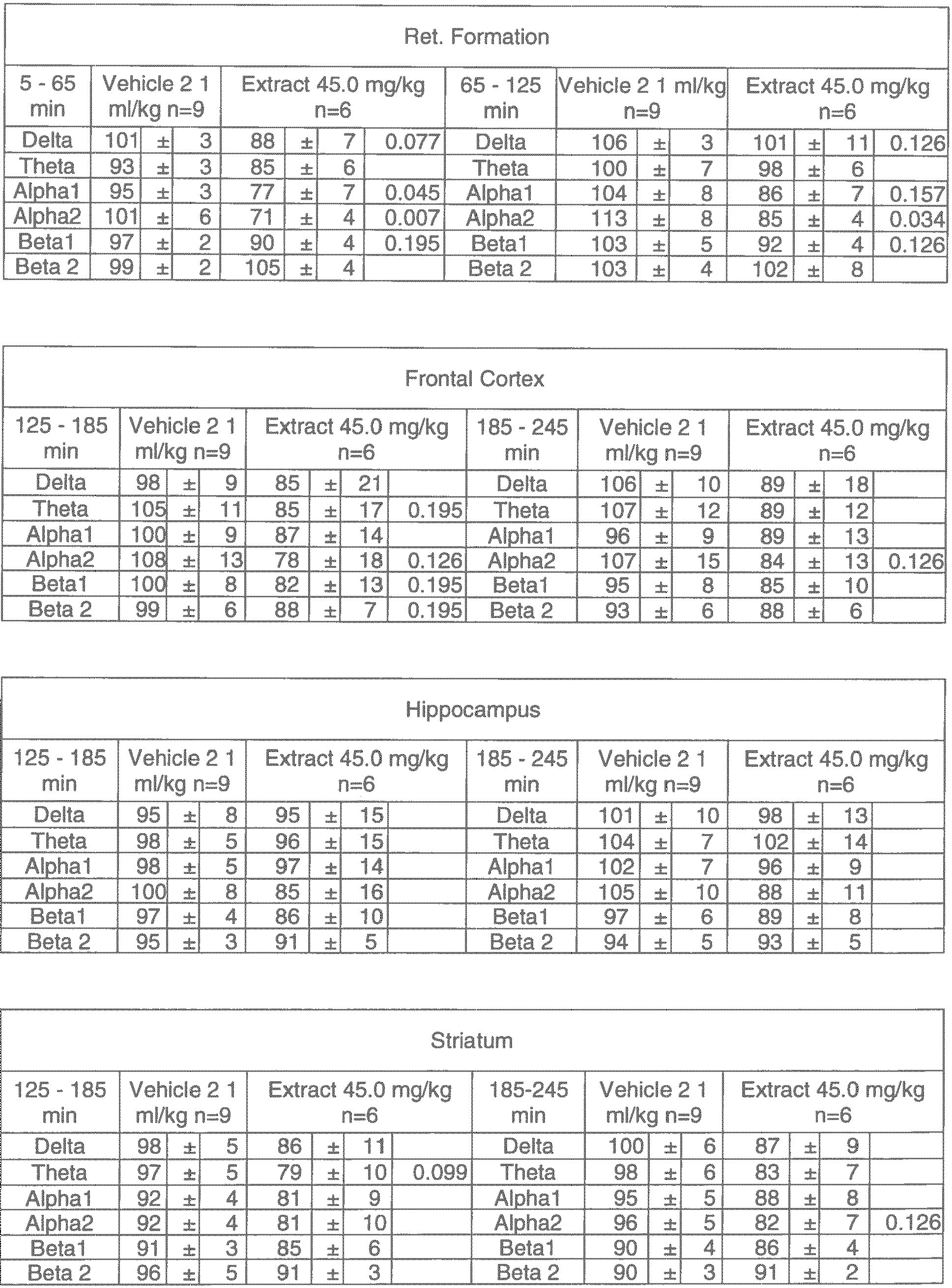

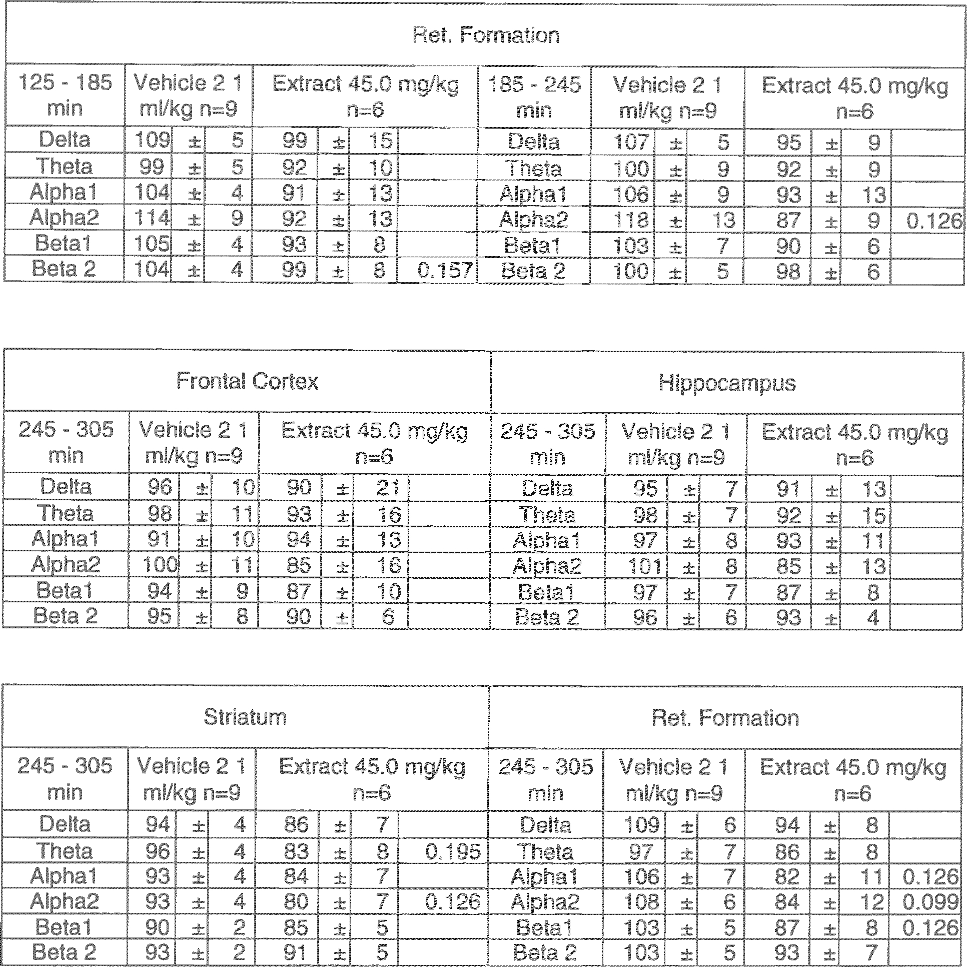

All four extracts were able to change the spectral power of electric brain activity in four areas in the freely moving rat.

The extracts of *Cistus incanus, Cichorium intybus,* and *Elymus repens* exert their action already with a dosage of 45.0 mg/kg. Strongest effects were observed in the presence of an extract of *Elymus repens.* Thus, the extracts are able to modulate different transmitter systems.

In summary, all four extracts were able to change the frequency pattern of the electric activity of four brain areas in the freely moving adult rat. Thus, the extracts are suitable in the treatment of the claimed neurodegenerative diseases.

## Claims

1. Composition for use in a method for inhibiting the reuptake of serotonin for the treatment and/or prophylaxis of a disorder connected to modified neurotransmission or a neurodegenerative disease, wherein the disorder or disease is selected from the group consisting of depressions, chronic pain, panic attacks and anxiety, said composition comprising an extract of at least one plant selected from *Cistus incanus* and *Cistus salviifolius,* wherein the solvent for the extract is water or a mixture of water with methanol or ethanol.

2. Composition for use in a method for inhibiting acetylcholinesterase for the treatment and/or prophylaxis of a disorder connected to modified neurotransmission or a neurodegenerative disease, wherein the disorder or disease is selected from the group consisting of Parkinson's disease and Alzheimer's disease, said composition comprising an extract of at least one plant selected from *Cistus incanus* and *Cistus salviifolius,* wherein the solvent for the extract is water or a mixture of water with methanol or ethanol.

3. The composition for use according to claim 1 or 2, wherein the extract is of the aerial parts of the plant.

4. The composition for use according to claim 3, wherein the aerial parts are selected from the leaves, twigs, blossoms and/or buds.

5. The composition for use according to any one of claims 1 to 4, wherein the composition is in liquid, dry or semi-solid form.

6. The composition for use according to any one of claims 1 to 5, wherein the composition is in the form of a tablet, capsule, powder, granulate, tea mixture, sirup, drops, tincture, emulsion, ointment, cream, gel, paste, infusion, inhalation solution or mouth spray.

## Patentansprüche

1. Zusammensetzung zur Verwendung in einem Verfahren zur Inhibierung der Wiederaufnahme von Serotonin zur Behandlung und/oder Prophylaxe einer Störung, die mit einer modifizierten Neurotransmission vebunden ist, oder einer neurodegenerativen Krankheit, wobei die Störung oder Krankheit ausgewählt ist aus der Gruppe bestehend aus Depressionen, chronischen Schmerzen, Panikattacken und Angstzuständen, wobei die Zusammensetzung einen Extrakt aus mindestens einer Pflanze, ausgewählt aus *Cistus incanus* und *Cistus salviifolius,* umfasst, wobei das Lösungsmittel für den Extrakt Wasser oder ein Gemisch aus Wasser mit Methanol oder Ethanol ist.

2. Zusammensetzung zur Verwendung in einem Verfahren zur Inhibierung von Acetylcholinesterase zur Behandlung und/oder Prophylaxe einer Störunug, die mit einer modifizierten Neurotransmission verbunden ist, oder einer neurodegenerativen Krankheit, wobei die Störung oder Krankheit ausgewählt ist aus der Gruppe bestehend aus der Parkinson-Krankheit und der Alzheimer-Krankheit, wobei die Zusammensetzung einen Extrakt aus mindestens einer Pflanze, ausgewählt aus *Cistus incanus* und *Cistus salviifolius,* umfasst, wobei das Lösungsmittel für den Extrakt Wasser oder ein Gemisch aus Wasser mit Methanol oder Ethanol ist.

3. Zusammensetzung zur Verwendung nach Anspruch 1 oder 2, wobei der Extrakt aus den oberirdischen Teilen der Pflanze besteht.

4. Zusammensetzung zur Verwendung nach Anspruch 3, wobei die oberirdischen Teile ausgewählt sind aus den Blättern, Zweigen, Blüten und/oder Knospen ausgewählt sind.

5. Zusammensetzung zur Verwendung nach einem der Ansprüche 1 bis 4, wobei die Zusammensetzung in flüssiger, trockener oder halbfester Form vorliegt.

6. Zusammensetzung zur Verwendung nach einem der Ansprüche 1 bis 5, wobei die Zusammensetzung in Form einer Tablette, Kapsel, eines Pulvers, eines Granulats, einer Teemischung, eines Sirups, von Tropfen, einer Tinktur, einer Emulsion, einer Salbe, einer Creme, eines Gels, einer Paste, eines Aufgusses, einer Inhalationslösung oder eines Mundsprays vorliegt.

## Revendications

1. Composition pour l'utilisation dans un procédé d'inhibition du reprélèvement de la sérotonine pour le traitement et/ou la prophylaxie d'un trouble lié à la neurotransmission modifiée ou d'une maladie neurodégénérative, le trouble ou la maladie étant sélectionné·e dans le groupe constitué des dépressions, de la douleur chronique, des crises de panique et de l'anxiété, ladite composition comprenant un extrait d'au moins une plante sélectionnée parmi *Cistus incanus* et *Cistus salviifolius,* le solvant pour l'extrait étant l'eau ou un mélange d'eau avec du méthanol ou de l'éthanol.

2. Composition pour l'utilisation dans un procédé d'inhibition de l'acétylcholinestérase pour le traitement et/ou la prophylaxie d'un trouble lié à la neurotransmission modifiée ou d'une maladie neurodégénérative, le trouble ou la maladie étant sélectionné·e dans le groupe constitué de la maladie de Parkinson et de la maladie d'Alzheimer, ladite composition comprenant un extrait d'au moins une plante sélectionnée parmi *Cistus incanus* et *Cistus salviifolius,* le solvant pour l'extrait étant l'eau ou un mélange d'eau avec du méthanol ou de l'éthanol.

3. Composition pour l'utilisation selon la revendication 1 ou 2, l'extrait provenant des parties aériennes de la plante.

4. Composition pour l'utilisation selon la revendication 3, les parties aériennes étant sélectionnées parmi les feuilles, les rameaux, les inflorescences et/ou les bourgeons.

5. Composition pour l'utilisation selon l'une quelconque des revendications 1 à 4, la composition se présentant sous une forme liquide, sèche ou semi-solide.

6. Composition pour l'utilisation selon l'une quelconque des revendications 1 à 5, la composition se présentant sous la forme d'un comprimé, d'une capsule, d'une poudre, d'un granulé, d'un mélange de thé, d'un sirop, de gouttes, de teinture, d'émulsion, d'onguent, de crème, de gel, de pâte, de perfusion, de solution pour inhalation ou de pulvérisation buccale.
